(19) Europäisches Patentamt
European Patent Office
Office européen des brevets

(11) Veröffentlichungsnummer: **0 212 600 B1**

# (12) EUROPÄISCHE PATENTSCHRIFT

(45) Veröffentlichungstag der Patentschrift: **27.11.91**

(51) Int. Cl.$^5$: **C07D 401/06**, A01N 43/40, A01N 43/50

(21) Anmeldenummer: **86111375.1**

(22) Anmeldetag: **18.08.86**

Die Akte enthält technische Angaben, die nach dem Eingang der Anmeldung eingereicht wurden und die nicht in dieser Patentschrift enthalten sind.

## (54) Neue Nitromethylen-Derivate.

(30) Priorität: **27.08.85 JP 186592/85**

(43) Veröffentlichungstag der Anmeldung:
**04.03.87 Patentblatt 87/10**

(45) Bekanntmachung des Hinweises auf die Patenterteilung:
**27.11.91 Patentblatt 91/48**

(84) Benannte Vertragsstaaten:
**AT BE CH DE FR GB IT LI NL**

(56) Entgegenhaltungen:
**EP-A- 136 636**
**EP-A- 0 154 178**
**EP-A- 0 163 855**
**DE-A- 2 732 660**

**PATENT ABSTRACTS OF JAPAN, Band 11, Nr. 3 (C-395)[2450], 7. Januar 1987; & JP-A-61 183 271 (NIPPON TOKUSHU NOYAKU SEIZO K.K.) 15-08-1986**

**Adv.Pestic.Chem:4th Congr:pest:chem.,Vol.2,206-217 (1979)**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Shiokawa, Kozo**
**210-6, Shukugawara Tama-ku**
**Kawasaki-shi Kanagawa-ken(JP)**
Erfinder: **Tsuboi, Shinichi**
**3-26-1, Hirayama**
**Hino-shi Tokyo(JP)**
Erfinder: **Kagabu, Shinzo**
**432-131-105, Terada-machi**
**Hachioji-shi Tokyo(JP)**
Erfinder: **Moriya, Koichi**
**39-15, Namiki-cho**
**Hachioji-shi Tokyo(JP)**
Erfinder: **Baasner, Bernd, Dr.**
**Hamberger Strasse 27d**
**W-5090 Leverkusen(DE)**

(74) Vertreter: **Schumacher, Günter, Dr. et al**
**c/o Bayer AG Konzernverwaltung RP Patentabteilung**
**W-5090 Leverkusen 1 Bayerwerk(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Nitromethylen-Derivate, Verfahren zu ihrer Herstellung sowie ihre Verwendung als Insektizide.

Es ist bereits bekannt geworden, daß bestimmte Nitromethylen-Derivate insektizide Wirkung besitzen (siehe die DE-OS 25 14 402).

Folgende Dokumente gemäß Artikel 54(3) EPÜ, welche ähnlich strukturierte Verbindungen offenbaren, sind bekannt: EP-A 0 192 060, EP-A 0 163 855 und EP-A 0 154 178. Weiterhin existieren folgende Publikationen mit strukturell verwandten Stoffen:EP-A 0 136 636, DE-A 2 732 660 und Adv.Pestic.Chem. 4th Int.Congr.Pest.Chem., Vol. 2, 206-217 (1979).

Nunmehr wurden neue Nitromethylen-Derivate der Formel (I)

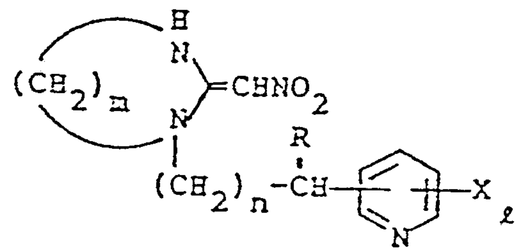

(I)

gefunden, in der

R ein Wasserstoff-Atom oder eine Alkyl-Gruppe mit 1 bis 4 Kohlenstoff-Atomen ist,
X eine durch Fluor, Chlor und/oder Brom substituierte $C_1$-$C_4$-Alkyl-Gruppe, eine durch Fluor und/oder Chlor substituierte $C_1$-$C_4$-Alkoxy-Gruppe, eine durch Fluor und/oder Chlor substituierte $C_1$-$C_4$-Alkylthio-Gruppe, eine Cyano-Gruppe oder eine Thiocyanato-Gruppe ist,
ℓ 1 oder 2 ist,
m 2 oder 3 ist und
n 0 ist.

Die Verbindungen der Formel (I) werden erhalten, wenn

(a) die Verbindungen der Formel (II)

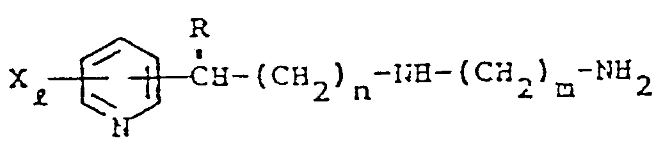

(II)

in der R, X, ℓ, m und n die im Vorstehenden angegebenen Bedeutungen haben, mit den Verbindungen der Formel (III)

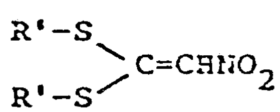

(III) ,

in der

R' eine niedere Alkyl-Gruppe oder eine Benzyl-Gruppe bezeichnet oder die beiden Gruppe R' zusammengenommen eine niedere Alkylen-Gruppe mit wenigstens zwei Kohlenstoff-Atomen bezeichnen und zusammen mit den ihnen benachbarten Schwefel-Atomen einen Ring bilden können,

in Gegenwart inerter Lösungsmittel umgesetzt werden,

oder

(b) die Verbindungen der Formel (IV)

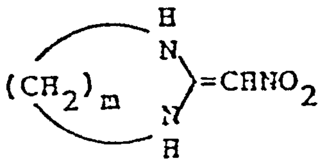

(IV)

,

in der m die im Vorstehenden angegebenen Bedeutungen hat, mit den Verbindungen der Formel (V)

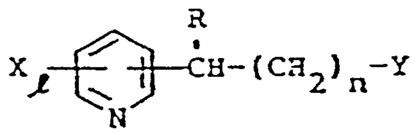

(V)

in der

R, X, ℓ und n — die im Vorstehenden angegebenen Bedeutungen haben und

Y — ein Halogen-Atom oder eine Gruppe der Formel $-OSO_2R''$ bezeichnet, worin R'' für eine niedere Alkyl-Gruppe oder eine Aryl-Gruppe steht,

in Gegenwart inerter Lösungsmittel, gegebenenfalls in Anwesenheit von Basen, umgesetzt werden.

Die neuen Nitromethylen-Derivate zeigen sehr starke insektizide Eigenschaften.

Überraschenderweise zeigen die Nitromethylen-Derivate gemäß der vorliegenden Erfindung eine wesentlich größere und sehr viel stärker herausragende insektizide Wirksamkeit als die nächstkommenden Verbindungen des oben genannten Standes der Technik.

Außerdem zeigen die erfindungsgemäßen Nitromethylen-Derivate auch eine bemerkenswerte insektizide Wirkung gegenüber Schadinsekten, insbesondere saugenden Insekten, wie sie typisch durch Insekten der Gattung Hemiptera repräsentiert werden, etwa Blattläuse, Laternenträger, Wanzen und Heuschrecken, die infolge einer Langzeit-Anwendung Resistenz gegen Insektizide vom Typ organischer Phosphate und Carbamate erworben haben.

Besonders bevorzugte Verbindungen der Formel (I) sind diejenigen, in denen

R — ein Wasserstoff-Atom oder eine Methyl-Gruppe ist,

X — eine durch Fluor substituierte $C_1$-$C_2$-Alkyl-Gruppe, eine durch Fluor substituierte $C_1$-$C_2$-Alkoxy-Gruppe, eine durch Fluor substituierte $C_1$-$C_2$-Alkylthio-Gruppe, eine Cyano-Gruppe oder eine Thiocyanato-Gruppe ist, ist,

ℓ — 1 oder 2 ist,

m — 2 oder 3 ist und

n — 0 ist.

Speziell erwähnt seien die folgenden Verbindungen:

1-(2-Cyano-5-pyridylmethyl)-2-(nitromethylen)tetrahydropyrimidin,
1-(2-Cyano-5-pyridylmethyl)-2-(nitromethylen)imidazolidin,
1-(2-Trifluoromethyl-5-pyridylmethyl)-2-(nitromethylen) imidazolidin,
1-(2-Trifluoromethoxy-5-pyridylmethyl)-2-(nitromethylen)tetrahydropyrimidin,
1-(2-Trifluoromethoxy-5-pyridylmethyl)-2-(nitromethylen) imidazolidin,
1-(2-Trifluoromethylthio-5-pyridylmethyl)-2-(nitromethylen)imidazolidin.

Die Verbindungen der Formel (I) gemäß der vorliegenden Erfindung können beispielsweise mittels der folgenden allgemeinen Verfahren hergestellt werden.

<u>Verfahren a)</u>

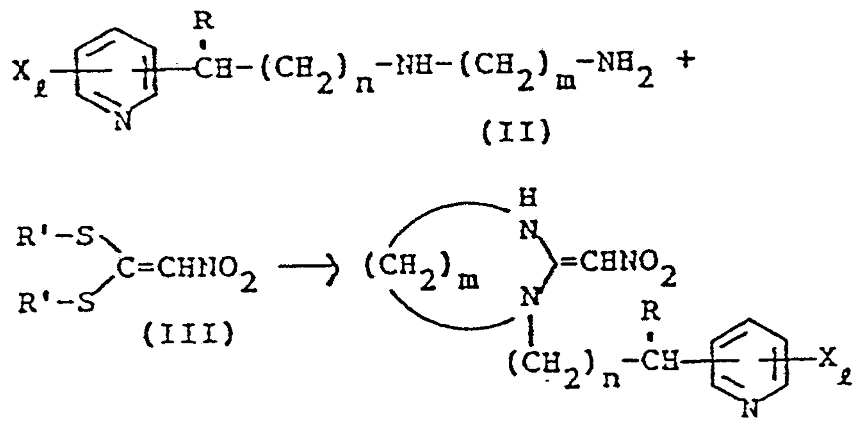

(II) (III) (I)

(In den Formeln haben R, X, ℓ, m, n und R' die oben angegebenen Bedeutungen).

Wenn beispielsweise N-(2-Cyano-5-pyridylmethyl)trimethylendiamin und 1-Nitro-2,2-bis(methylthio)-ethylen als Ausgangsstoffe in dem vorstehenden Verfahren eingesetzt werden, wird die Reaktion durch das folgende Schema dargestellt.

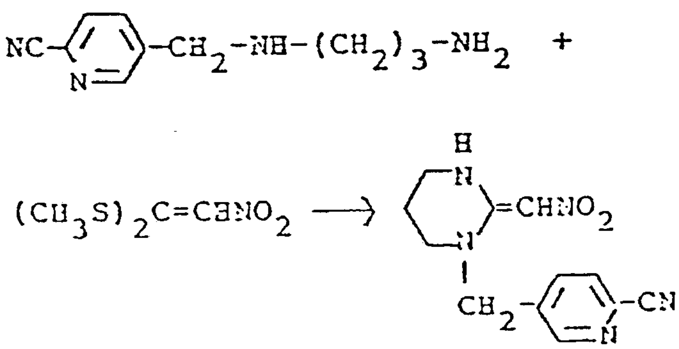

Verfahren b)

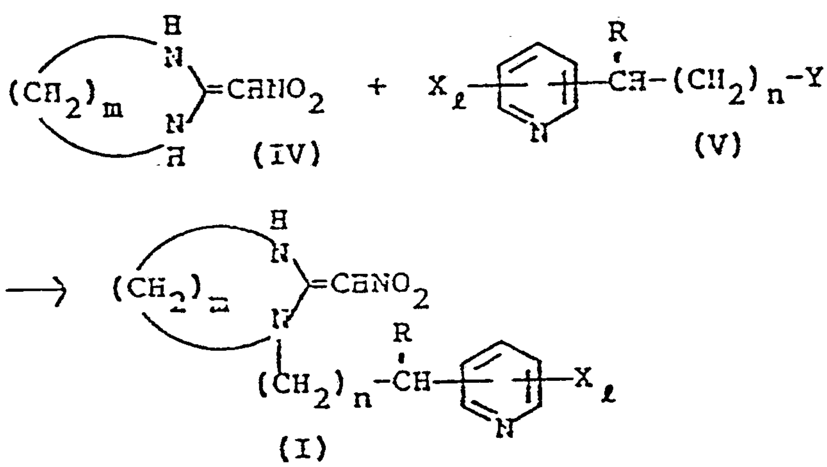

(IV) (V) (I)

(In den Formeln haben R, X, ℓ, m, n und Y die oben angegebenen Bedeutungen).

Wenn 2-Nitromethylenimidazolidin und 2-Cyano-5-pyridylmethylchlorid als Ausgangsstoffe in dem Verfahren b) eingesetzt werden, wird die Reaktion durch das folgende Reaktionsschema dargestellt.

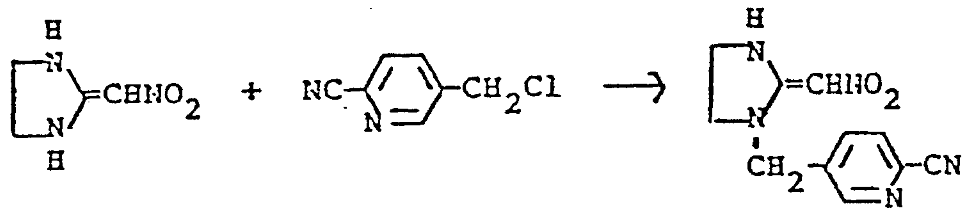

Die Verbindungen der Formel (I), in denen X eine Halogenoalkyl-Gruppe, eine Halogenoalkoxy-Gruppe oder eine Halogenoalkylthio-Gruppe ist und $\ell$ 1 ist, können mittels des Verfahrens c) zusätzlich zu den Verfahren a) und b) hergestellt werden.

Verfahren c)

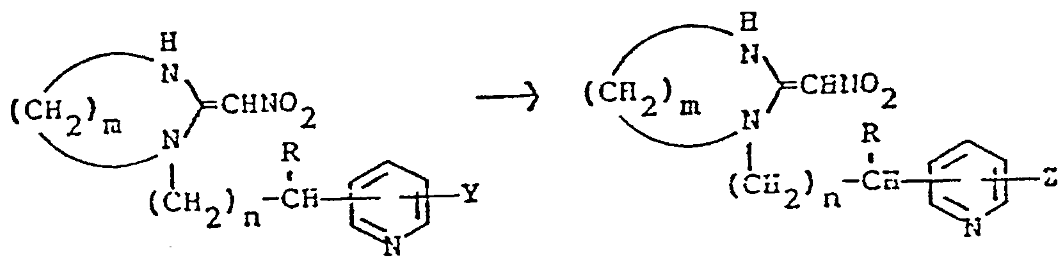

(I') (I")

(In den Formeln haben R, m und n die oben angegebenen Bedeutungen, Y bezeichnet ein Halogen-Atom und Z bezeichnet eine Halogenoalkyl-, Halogenoalkoxy- oder Halogenoalkylthio-Gruppe).

Wenn 1-(2-Bromo-5-pyridylmethyl)-2-(nitromethylen)imidazolidin mit Trifluoromethyl-kupfer in dem vorstehenden Verfahren umgesetzt wird, wird die Reaktion durch das folgende Schema veranschaulicht.

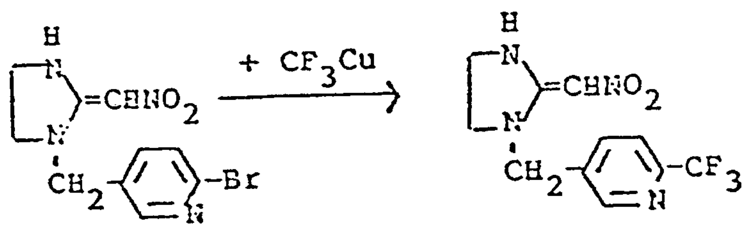


Die Verbindung der Formel (I') in Verfahren c) ist die in den JP-Patentanmeldungen 72 966/1984 und 132 943/1984 der Anmelderin der vorliegenden Anmeldung beschriebene Verbindung.

In dem Verfahren c) lassen sich die halogensubstituierten Verbindungen der Formel (I') durch Erhitzen mit, beispielsweise, Trifluoromethyl-kupfer, wie oben gezeigt wurde, in ein trifluormethylsubstituiertes Produkt überführen (durch Anwenden der in der JP-OS 22 371/1979 und der auf Seite 989 der Pre-Prints of the 50th Spring Meeting of the Chemical Society of Japan beschriebenen Methoden).

Das Halogenoalkoxy- oder das Halogenoalkylthio-Substitutionsprodukt kann leicht durch Umsetzung der halogensubstituierten Verbindung (I') mit einem Halogenoalkoxid oder einem Halogenomercaptid hergestellt werden.

In dem Verfahren a) bezeichnen die Verbindungen der Formel (II) als Ausgangsstoffe Verbindungen, für die die vorstehend gegebenen Definitionen für R, X, $\ell$, m und n gelten, und vorzugsweise sind die Bedeutungen von R, X, $\ell$, m und n gleichlautend mit den oben als bevorzugt genannten Bedeutungen.

Die der Formel (II) entsprechenden Verbindungen waren vor dem Anmeldezeitpunkt der vorliegenden Anmeldung nicht bekannt. Zu speziellen Beispielen für dieselben zählen

N-(2-Fluoromethoxy-5-pyridylmethyl)-,

N-(2-Difluoromethoxy-5-pyridylmethyl)-,

N-(2-Trifluoromethoxy-5-pyridylmethyl)-,
N-[1-(2-Trifluoromethoxy-5-pyridyl)ethyl]-,
N-[2-(2-Fluoroethoxy)-5-pyridylmethyl]-,
N-[2-(2-Chloroethoxy)-5-pyridylmethyl]-,
N-[2-(2-Bromoethoxy)-5-pyridylmethyl]-,
N-[2-(2,2,2-Trifluoroethoxy)-5-pyridylmethyl]-,
N-[2-(1,1,2,2-Tetrafluoroethoxy)-5-pyridylmethyl]-,
N-[2-(1,1,1,3,3,3-Hexafluoro-2-propoxy)-5-pyridylmethyl]-,
N-(2-Difluormethylthio-5-pyridylmethyl)-,
N-(2-Trifluoromethylthio-5-pyridylmethyl)-,
N-[2-(2,2,2-Trifluoroethylthio)-5-pyridylmethyl]-,
N-(5-Trifluoromethylthio-2-pyridylmethyl)-,
N-[2-(2-Fluoroethylthio)-5-pyridylmethyl]-,
N-(2-Cyano-5-pyridylmethyl)-,
N-(2-Thiocyanato-5-pyridylmethyl)-,
N-[1-(2-Cyano-5-pyridyl)ethyl]-,
N-(2-Fluoromethyl-5-pyridylmethyl)-,
N-(2-Difluoromethyl-5-pyridylmethyl)-,
N-(2-Trifluoromethyl-5-pyridylmethyl)-,
N-(2-Chlorodifluoromethyl-5-pyridylmethyl)-,
N-[1-(2-Trifluoromethyl-5-pyridyl)ethyl]-,
N-(2-Bromodifluoromethyl-5-pyridylmethyl)-,
N-(5-Trifluoromethyl-2-pyridylmethyl)-,
N-[2-(2-Fluoroethyl)-5-pyridylmethyl]-,
N-[2-(2-Chloroethyl)-5-pyridylmethyl]-,
N-[2-(2,2,2-Trifluoroethyl)-5-pyridylmethyl]-,
N-[2-(1,1,2,2-Tetrafluoroethyl)-5-pyridylmethyl]- und
N-[2-(1,1,2,2,2-Pentafluoroethyl)-5-pyridylmethyl]ethylendiamin und -trimethylendiamin.

Die oben beispielhaft aufgeführten Ethylendiamine und Trimethylendiamine der Formel (II) können mittels des folgenden Verfahrens hergestellt werden.

Verfahren α)

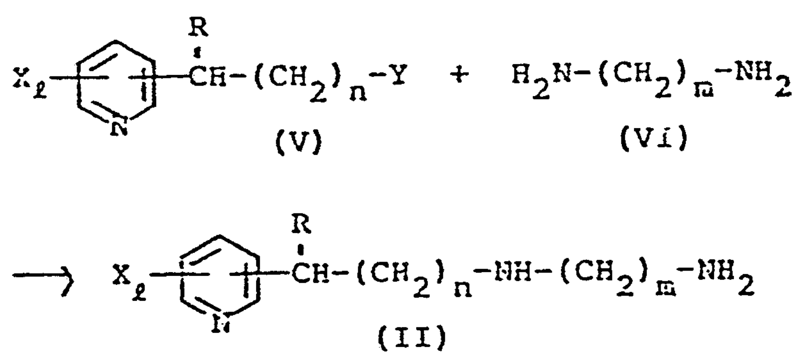

(V) (VI)

(II)

(In den Formeln haben R, X, $\ell$, m, n und Y die oben angegebenen Bedeutungen).

Die Verbindungen der Formel (V) als Ausgangsstoffe in dem Verfahren α sind die gleichen wie die Ausgangsstoffe in dem Verfahren b), die im einzelnen hiernach noch beschrieben werden.

Die Verbindungen der Formel (VI) sind in der DE-OS 27 32 660 und der FR-PS 1 499 785 beschrieben. Ihre speziellen Beispiele sind Ethylendiamin und Trimethylendiamin (auch als 1,3-Diaminopropan bekannt).

In der Praxis des Verfahrens α lassen sich die gewünschten Verbindungen der Formel (II) leicht dadurch erhalten, daß man die Reaktion in einem inerten Lösungsmittel durchführt, wie es beispielhaft für das Verfahren a) genannt ist, das im einzelnen hiernach noch beschrieben wird.

Das Verfahren α kann in einfacher Weise praktisch durchgeführt werden, indem eine überschüssige Stoffmenge, beispielsweise etwa 5 Mol, der Verbindung der allgemeinen Formel (VI) mit 1 Mol der Verbindung der allgemeinen Formel (V) bei einer Reaktionstemperatur von gewöhnlich 0 °C bis 50 °C

umgesetzt wird.

Die Verbindungen der Formel (II), in denen n Null ist, können auch mittels des folgenden alternativen Verfahrens hergestellt werden.

Verfahren β)

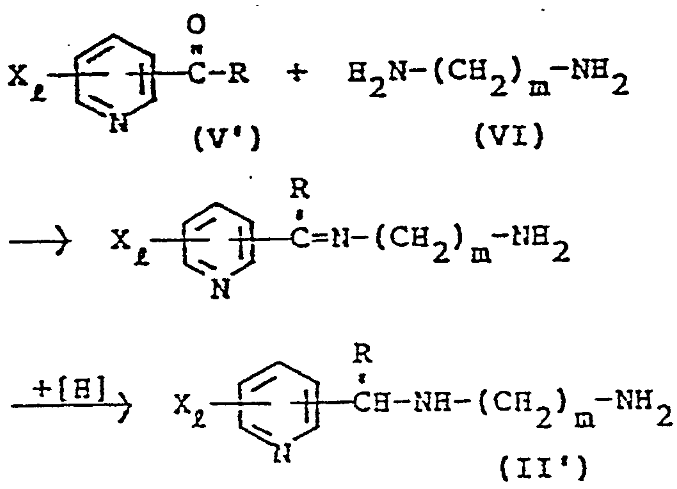
(V') (VI) (II')

(In den Formeln haben X, R, ℓ und m die oben angegebenen Bedeutungen).

In dem Verfahren β) sind die meisten der Verbindungen der Formel (V') in der JP-Patentanmeldung 18 628/1985 der Anmelderin der vorliegenden Anmeldung beschrieben, und einige von ihnen sind in J. Med. Chem. 13, Seiten 1124-1130, beschrieben. Im allgemeinen können diese substituierten Pyridincarbaldehyde mittels der bekannten, in J. Org. Chem. 26, Seiten 4912-4914, beschriebenen Methode oder durch Ausnutzung der Reaktion der Reduktion der entsprechenden Pyridincarbonsäuren oder ihrer Derivate zu Aldehyden (Org. Reaction, Vol. 8, Seiten 218-257) synthetisiert werden. Wie anhand des Reaktionsschemas gemäß dem Verfahren β) gezeigt ist, können die Verbindungen der Formel (II') dadurch synthetisiert werden, daß der Pyridincarbaldehyd oder das Pyridinalkylketon der Formel (V') mit der Verbindung der Formel (VI) zu einem Imin umgesetzt wird und dieses mit einem Reduktionsmittel wie Natriumborhydrid ($NaBH_4$) umgesetzt wird.

Die Verbindungen der Formel (II), in denen R Wasserstoff ist und n Null ist, können auch mittels des folgenden alternativen Verfahrens hergestellt werden.

Verfahren γ)

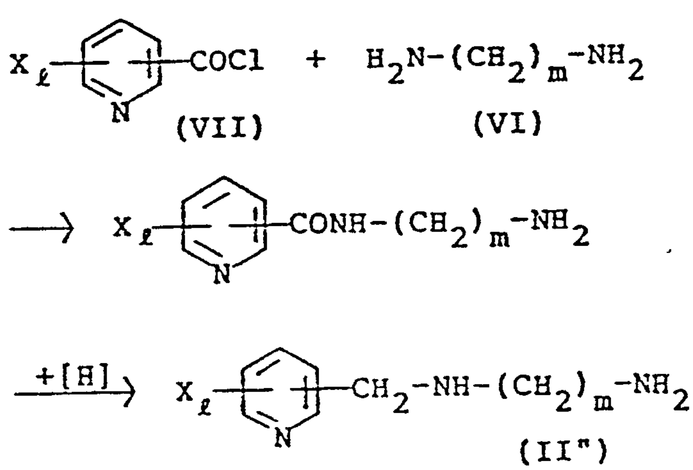
(VII) (VI) (II")

(In den Formeln haben x, ℓ und m die oben angegebenen Bedeutungen).

Wie oben schematisch dargestellt ist, kann die Verbindung der Formel (II") durch Reaktion des Pyridylcarbonylchlorids der Formel (VII) mit der Verbindung der allgemeinen Formel (VI) unter Bildung eines Nicotinamids oder Picolinamids und Reaktion des letzteren mit einem Reduktionsmittel wie Lithiumaluminiumhydrid ($LiAlH_4$) synthetisiert werden.

Die Verbindungen der Formel (II), in denen R Wasserstoff ist, n Null ist und m 3 ist, können auch mittels des folgenden alternativen Verfahrens hergestellt werden.

Verfahren δ)

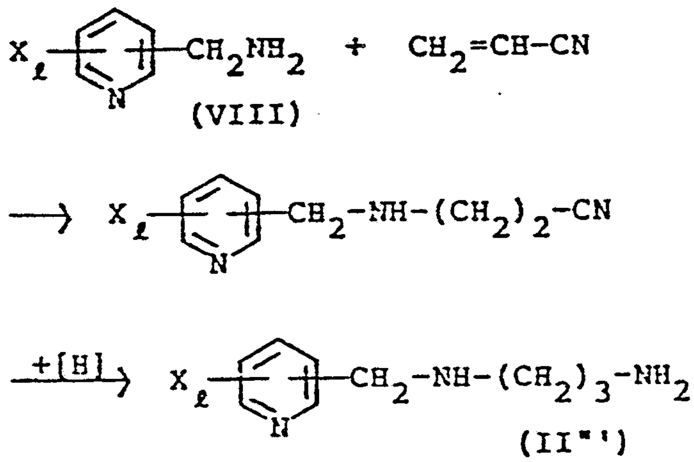

(VIII)

(II''')

(In den Formeln haben X und ℓ die oben angegebenen Bedeutungen).

Wie oben schematisch dargestellt ist, können die Verbindung der Formel (II''') durch Additionsreaktion von Acrylnitril mit den Verbindungen der Formel (VIII) und Reduktion des Addukts in gleicher Weise wie in Verfahren β) synthetisiert werden.

Die Verbindungen der Formel (II), in denen m 2 ist, können auch alternativ mittels des folgenden Verfahrens hergestellt werden.

Verfahren ε)

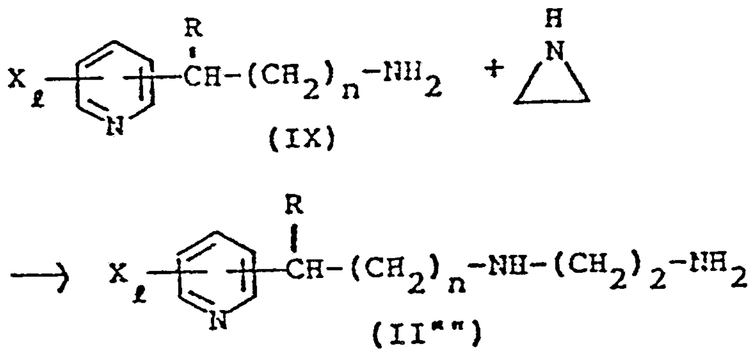

(IX)

(II'''')

(In den Formeln haben R, X, ℓ und n die oben angegebenen Bedeutungen).

Wie oben schematisch dargestellt ist, können die Verbindung der Formel (II'''') durch Reaktion des Pyridylalkylamins der Formel (IX) mit Ethylenimin synthetisiert werden.

Die Verbindungen der Formel (III) als Ausgangsstoffe in dem Verfahren a) sind bekannte Verbindungen, die beispielsweise in Chem. Ber. 100, Seiten 591-604, beschrieben sind. Zu speziellen Beispielen für dieselben zählen

1-Nitro-2,2-bis(methylthio)ethylen,

1-Nitro-2,2-bis(ethylthio)ethylen,

1-Nitro-2,2-bis(benzylthio)ethylen und
2-Nitromethylen-1,3-dithiolan.

Die Verbindungen der Formel (IV) als Ausgangsstoffe in dem Verfahren b) bedeuten Verbindungen, für die die vorstehend gegebene Definition für m gilt, und vorzugsweise sind die Bedeutungen von m gleichlautend mit den oben als bevorzugt genannten Bedeutungen.

Die Verbindungen der Formel (IV) sind beispielsweise in Chem. Ber. 100, Seiten 591-604, beschrieben. Spezielle Beispielen für dieselben sind
2-Nitromethylenimidazolidin und
2-Nitromethylentetrahydropyrimidin.

Die Verbindungen der Formel (V) als Ausgangsstoffe in Verfahren b) bezeichnen Verbindungen, für die die vorstehend gegebenen Definitionen für R, X, $\ell$, n und Y gelten, und vorzugsweise sind die Bedeutungen von R, X, $\ell$ und n gleichlautend mit den oben als bevorzugt genannten Bedeutungen. Y bezeichnet ein Chlor- oder Brom-Atom.

Gemäß der vorliegenden Erfindung können die Verbindungen der Formel (V) als Ausgangsstoffe mittels eines bekannten Verfahrens hergestellt werden.

Zu speziellen Beispielen für die Verbindungen der Formel (V) zählen
(2-Fluoromethoxy-5-pyridylmethyl-,
(2-Difluoromethoxy-5-pyridylmethyl-,
(2-Trifluoromethoxy-5-pyridylmethyl-,
1-(2-Trifluoromethoxy-5-pyridyl)ethyl-,
2-(2-Fluoroethoxy)-5-pyridylmethyl-,
2-(2-Chloroethoxy)-5-pyridylmethyl-,
2-(2-Bromoethoxy)-5-pyridylmethyl-,
2-(2,2,2-Trifluoroethoxy)-5-pyridylmethyl-,
2-(1,1,2,2-Tetrafluoroethoxy)-5-pyridylmethyl-,
2-(1,1,1,3,3,3-Hexafluoro-2-propoxy)-5-pyridylmethyl-,
2-Difluoromethylthio-5-pyridylmethyl-,
2-Trifluoromethylthio-5-pyridylmethyl-,
2-(2,2,2-Trifluoromethylthio)-5-pyridylmethyl-,
5-Trifluoromethylthio-2-pyridylmethyl-,
2-(2-Fluoroethylthio)-5-pyridylmethyl-,
2-Cyano-5-pyridylmethyl-,
1-(2-Cyano-5-pyridyl)ethyl-,
1-(2-Thiocyanato-5-pyridyl)methyl-,
2-Fluoromethyl-5-pyridylmethyl-,
2-Difluoromethyl-5-pyridylmethyl-,
2-Trifluoromethyl-5-pyridylmethyl-,
2-Chlorodifluoromethyl-5-pyridylmethyl-,
1-(2-Trifluoromethyl-5-pyridyl)ethyl-,
2-Bromodifluoromethyl-5-pyridylmethyl-,
5-Trifluoromethyl-2-pyridylmethyl-,
2-(2-Fluoroethyl)-5-pyridylmethyl-,
2-(2-Chloroethyl)-5-pyridylmethyl-,
2-(2,2,2-Trifluoroethyl)-5-pyridylmethyl-,
2-(1,1,2,2-Tetrafluoroethyl)-5-pyridylmethyl- und
2-(1,1,2,2,2-Pentafluoroethyl)-5-pyridylmethylchlorid oder bromid.

Die hiervor beispielhaft aufgeführten Chloride lassen sich in einfacher Weise nach einer gebräuchlichen Methode der Chlorierung des entsprechenden Alkohols, beispielsweise mit Thionylchlorid, synthetisieren. Die Halogenide lassen sich auch synthetisieren durch Halogenierung der seitenständigen Methyl-Gruppe mit Hilfe eines Halogenierungsmittels wie N-Bromsuccinimid oder N-Chlor succinimid.

Einige trifluoromethyl- oder trifluoromethoxy-substituierte Pyridylmethylalkohole sind in J. Med. Chem. 13, Seiten 1124-1130, beschrieben. 5-Trifluoromethyl-2-pyridylmethylalkohol kann nach dieser Synthesetechnik dadurch hergestellt werden, daß 2-Methyl-5-trifluoromethylpyridin, das durch Umsetzung von 6-Methylnicotinsäure mit Flußsäure oder Schwefeltetrafluorid erhalten wurde, in ein N-Oxid überführt und das N-Oxid einer Umlagerungsreaktion unterworfen wird.

Diese Reaktion läßt sich auch anwenden auf die Synthese von 5-Methyl-2-trifluoromethylpyridin aus 5-Methylpicolinsäure. Das neue 2-Trifluoromethyl-5-pyridylmethylbromid (oder -chlorid) als gewünschtes Ausgangsmaterial kann durch Behandeln des oben genannten 5-Methyl-2-trifluoromethylpyridins mit N-Bromosuccinimid oder N-Chlorosuccinimid zur Monohalogenierung der Methyl-Gruppe in der 5-Position synthetisiert werden.

2-Trifluoromethoxy-5-pyridylmethylbromid (oder -chlorid) kann in gleicher Weise durch Reaktion von 5-Methyl-2-trifluoromethoxypyridin, das aus 2-Hydroxy-5-methylpyridin erhalten wurde, mit N-Bromosuccinimid oder N-Chlorosuccinimid gewonnen werden.

Da Halogen in der ortho-Stellung des Pyridin-Rings aktiv ist, kann eine 6-Halogenoalkoxy-nicotinsäure beispielsweise dadurch synthetisiert werden, daß 6-Chloronicotinsäure mit einem Überschuß an Natriumalkoxid umgesetzt wird. Die Reduktion des Produkts liefert den 2-Halogenoalkoxy-5-pyridylmethylalkohol als Ausgangsstoff.

Alternativ kann das halogenoalkylsubstituierte Pyridin direkt durch Kondensationsreaktion von Methylbutadien mit einem Halogenoalkannitril wie Trifluoroacetonitril synthetisiert werden. Beispielsweise läßt sich 5-Methyl-2-trifluoromethylpyridin erhalten aus Methylbutadien und Trifluoroacetonitril (durch Anwenden der in J. Amer. Chem. Soc. 78, Seiten 978-979, und J. Org. Chem. 29, Seiten 569-571, beschriebenen Methoden).

Im Hinblick auf halogenoalkylthiosubstituierte Pyridine kann das als Ausgangsstoffe dienende Pyridylmethylchlorid (oder -bromid) beispielsweise durch Reaktion von 5-Methylpyridin-2-thion (beschrieben in Synth. Commun. 11, Seiten 273-280) mit einem geeigneten Alkylierungsmittel in Gegenwart eines Alkali unter Bildung eines 2-Halogenoalkylthio-5-methylpyridins und Behandeln desselben mit N-Bromosuccinimid oder N-Chlorosuccinimid erhalten werden.

Bei der praktischen Durchführung des Verfahrens a) können sämtliche inerten organischen Lösungsmittel als geeignete Verdünnungsmittel verwendet werden.

Beispiele für solche Verdünnungsmittel umfassen Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Das vorstehende Verfahren kann in einem weiten Temperaturbereich durchgeführt werden, beispielsweise zwischen etwa -20 °C und dem Siedepunkt der Mischung, vorzugsweise zwischen etwa 0 °C und etwa 100 °C. Zweckmäßigerweise wird die Reaktion unter normalem Atmosphärendruck durchgeführt, jedoch ist es auch möglich, unter erhöhtem oder vermindertem Druck zu arbeiten.

Bei der Durchführung des vorstehenden Verfahrens werden 1 mol der Verbindungen der Formel (II) und 1 bis etwa 1,2 mol, vorzugsweise 1 bis etwa 1,1 mol, der Verbindungen der Formel (III) beispielsweise in einem inerten Lösungsmittel wie einem Alkohol (z.B. Methanol oder Ethanol) umgesetzt, bis die Bildung von Mercaptan beendet ist. Dies vermag die gewünschten neuen Verbindungen der Formel (I) zu liefern.

Geeignete Verdünnungsmittel bei der praktischen Durchführung des Verfahrens b) können sämtliche der inerten organischen Lösungsmittel sein, die oben beispielhaft für das Verfahren a) angegeben sind, wobei Wasser und Alkohole ausgenommen sind. Weiterhin sind Hydride wie Natriumhydrid und Kaliumhydrid als Beispiele für die Basen zu nennen.

Das Verfahren b) kann in einem weiten Temperaturbereich, beispielsweise bei einer Temperatur zwischen etwa 0 °C und etwa 100 °C, vorzugsweise zwischen etwa 10 °C und etwa 50 °C, durchgeführt werden. Die Reaktion wird vorzugsweise unter normalem Atmosphärendruck durchgeführt, jedoch kann sie auch unter Bedingungen erhöhten oder verminderten Drucks durchgeführt werden.

Bei der praktischen Durchführung des vorstehenden Verfahrens lassen sich die gewünschten Verbindungen der Formel (I) dadurch erhalten, daß 1 mol der Verbindungen der Formel (IV) mit etwa 1 bis etwa 1,2 mol der Verbindungen der Formel (V) in Anwesenheit von etwa 1,1 bis 1,2 mol Natriumhydrid als Base in einem inerten Lösungsmittel wie Dimethylformamid umgesetzt wird. In dem Verfahren b) wird bevorzugt, daß die Verbindungen der Formel (IV) vorher durch Natriumhydrid in ein Natrium-Salz umgewandelt werden. Im Hinblick auf die Eigenschaften des Natriumhydrids wird zweckmäßigerweise eine solche Reaktion in einer Stickstoff-Atmosphäre durchgeführt.

Die Verbindungen der Formel (I) gemäß der vorliegenden Erfindung umfassen auch Tautomere, wie sie durch die folgenden Formeln dargestellt sind.

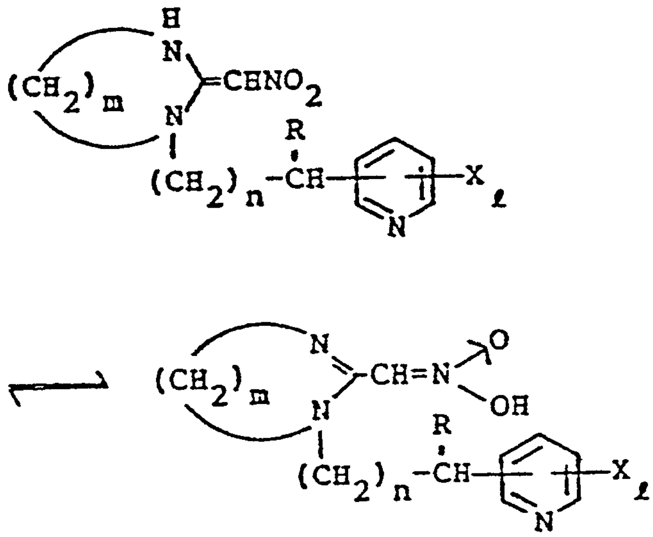

Die Verbindungen der Formel (I) gemäß der vorliegenden Erfindung können in Form von Salzen existieren. Beispiele für solche Salze umfassen anorganische Salze, Sulfonsäure-Salze, Salze organischer Säuren und Metall-Salze. Demgemäß ist der Begriff Nitromethylen-Derivate der Formel (I) so zu verstehen, daß er die Salze mit einschließt.

Die Verbindungen der Formel (I) gemäß der vorliegenden Erfindung zeigen starke insektizide Aktivität und können aus diesem Grunde als Insektizide eingesetzt werden. Die durch die Formel (I) bezeichneten aktiven Verbindungen der vorliegenden Erfindung zeigen einen genauen Bekämpfungseffekt gegen Schadinsekten, ohne Phytotoxizität bei Kulturpflanzen zu verursachen. Weiterhin können die Verbindungen der vorliegenden Erfindung zur Bekämpfung und Ausrottung eines weiten Bereichs von Schädlingen verwendet werden, darunter saugende Insekten, beißende Insekten und andere Pflanzenparasiten, Schädlinge in Getreidevorräten und gesundheitsgefährdende Schädlinge.

Beispiele für die zu bekämpfenden Schädlinge sind im Folgenden angegeben.

Insekten der Ordnung Coleoptera

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus;

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,

Agrotis fucosa,
Galleria mellonella,
Plutella maculipennis und
Phyllocnistis citrella;

<u>Insekten der Ordnung Hemiptera</u>

Nephotettix cincticeps,
Nilaparvata lugens,
Pseudococcus comstocki,
Unaspis yanonensis,
Myzus persicae,
Aphis pomi,
Aphis gossypii
Rhopalosiphum pseudobrassicae,
Stephanitis nashi,
Nazara spp.,
Cimex lectularius,
Trialeurodes vaporariorum und
Psylla spp.;

<u>Insekten der Ordnung Orthoptera</u>

Blatella germanica,
Periplaneta americana,
Gryllotalpa africana und
Locusta migratoria migratoriodes;

<u>Insekten der Ordnung Isoptera</u>

Deucotermes speratus und
Coptotermes formosanus;

<u>Insekten der Ordnung Diptera</u>

Musca domestica,
Aedes aegypti,
Hylemia platura
Culex pipiens,
Anopheles sinensis und
Culex tritaeniorhynchus.

Auf dem Gebiet der Veterinärmedizin sind die neuen Verbindungen gemäß der vorliegenden Erfindung gegenüber verschiedenen schädlichen Tierparasiten (inneren und äußeren Parasiten) wie Zecken, Insekten und Würmern wirksam.

Spezielle Beispiele für solche Tierparasiten sind Insekten wie
Gastrophilus spp.,
Stomoxys spp.,
Trichodectes spp.,
Rhodnius spp. und
Ctenocephalides canis.

In der vorliegenden Erfindung werden Substanzen mit pestizider Aktivität gegen sämtliche dieser Schädlinge gelegentlich als Insektizide bezeichnet.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolygycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie AlizarinFarbstoffe, Azo-Farbstoffe oder MetallphthalocyaninFarbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren.

Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen.

<u>Herstellungsbeispiele</u>

Beispiel 1

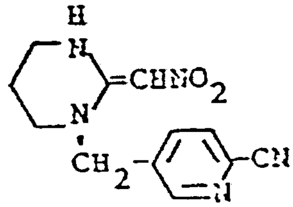

(Verbindung Nr. 1)

Eine Mischung aus N-(2-Cyano-5-pyridylmethyl)trimethylendiamin (1,9 g), 1-Nitro-2,2-bis(methylthio)-ethylen (1,6 g) und Ethanol (30 ml) wurde unter Rühren 5 h zum Rückfluß erhitzt. Die Reaktionsmischung wurde auf Raumtemperatur abgekühlt. Die abgeschiedenen Kristalle wurden durch Filtration gesammelt und mit Ethanol gewaschen, wonach blaßgelbes 1-(2-Cyano-5-pyridylmethyl)-2-(nitromethylen)-tetrahydropyrimidin (1,6 g) erhalten wurde; Schmp. 214-216° C.

Beispiel 2

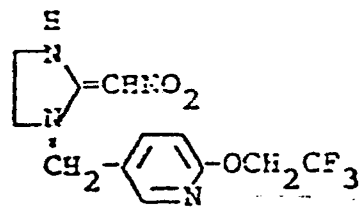

(Verbindung Nr. 2)

2,2,2-Trifluoroethanol (3,6 g) wurde in Toluol (20 ml) gelöst, und Natriumhydrid (0,7 g) wurde zur Herstellung des Natriumsalzes von 2,2,2-Trifluoroethanol hinzugefügt. Dann wurden 3 g 1-(2-Bromo-5-pyridylmethyl)-2-(nitromethylen)imidazolidin (die in der JP-Patentanmeldung 132 943/1984 beschriebene Verbindung) hinzugegeben, und die Mischung wurde 8 h auf 80° C erhitzt. Das Toluol wurde unter vermindertem Druck abdestilliert, und der Rückstand wurde in 20 ml Eiswasser gegossen und dann neutralisiert. Die wäßrige Lösung wurde mit Dichloromethan extrahiert, und der Extrakt wurde durch Silicagel-Säulenchromatographie gereinigt, wonach 1-[-2-(2,2,2-Trifluoroethoxy)-5-pyridylmethyl]-2-(nitromethylen)imidazolidin erhalten wurde; Schmp. 132-135° C.

Die nachstehende Tabelle 1 zeigt Beispiele für die Verbindungen der allgemeinen Formel (I) gemäß der vorliegenden Erfindung, die nach der Methode von Beispiel 1 oder 2 hergestellt wurden.

Tabelle 1

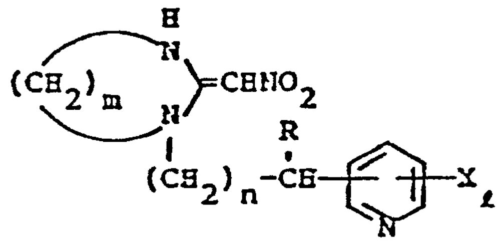

| Verbindung Nr. | m | n | R | Bindungsposition am Pyridin-Ring | $X_\ell$ mit Bindungsposition am Pyridin-Ring | |
|---|---|---|---|---|---|---|
| 3 | 2 | 0 | H | 5- | $2-OCH_2F$ | |
| 4 | 2 | 0 | H | 5- | $2-OCHF_2$ | |
| 5 | 2 | 0 | H | 5- | $2-OCF_3$ | |
| 6 | 3 | 0 | H | 5- | $2-OCF_3$ | |
| 7 | 2 | 0 | $-CH_3$ | 5- | $2-OCF_3$ | |
| 8 | 3 | 0 | H | 5- | $2-OCH_2CH_2F$ | |
| 9 | 2 | 0 | H | 5- | $2-OCH_2CH_2Cl$ | |
| 10 | 2 | 0 | H | 5- | $2-OCH_2CH_2Br$ | |
| 11 | 3 | 0 | H | 5- | $2-OCH_2CF_3$ | |
| 12 | 2 | 0 | H | 5- | $2-OCF_2CHF_2$ | |
| 13 | 2 | 0 | H | 5- | $2-OCH(CF_3)_2$ | |
| 14 | 3 | 0 | H | 5- | $2-SCF_3$ | |
| 15 | 2 | 0 | H | 5- | $2-SCF_3$ | |
| 16 | 2 | 1 | H | 5- | $2-SCH_2CF_3$ | |
| 17 | 2 | 0 | H | 2- | $5-SCF_3$ | |
| 18 | 2 | 0 | H | 5- | $2-SCH_2CH_2F$ | |
| 19 | 2 | 0 | H | 5- | 2-CN | Schmp. 155-158°C |
| 20 | 2 | 0 | $-CH_3$ | 5- | 2-CN | |

| Verbindung Nr. | m | n | R | Bindungsposition am Pyridin-Ring | $X_\ell$ mit Bindungsposition am Pyridin-Ring | |
|---|---|---|---|---|---|---|
| 21 | 2 | 0 | H | 5- | $2-CH_2F$ | |
| 22 | 2 | 0 | H | 5- | $2-CHF_2$ | |
| 23 | 3 | 0 | H | 5- | $2-CHF_2$ | Schmp. |
| 24 | 2 | 0 | H | 5- | $2-CF_3$ | 158-159°C |
| 25 | 3 | 0 | H | 5- | $2-CF_3$ | |
| 26 | 2 | 0 | $-CH_3$ | 5- | $2-CF_3$ | |
| 27 | 2 | 0 | H | 2- | $5-CF_3$ | |
| 28 | 2 | 0 | H | 5- | $2-CH_2CH_2F$ | |
| 29 | 2 | 0 | H | 5- | $2-CH_2CH_2Cl$ | |
| 30 | 2 | 0 | H | 5- | $2-CH_2CF_3$ | |
| 31 | 2 | 0 | H | 5- | $2-CF_2CHF_2$ | |
| 32 | 2 | 0 | H | 5- | $2-CF_2CF_3$ | |
| 33 | 2 | 1 | H | 5- | $2-CF_3$ | |
| 34 | 3 | 0 | H | 2- | $5-CF_3$ | |
| 35 | 2 | 0 | H | 5- | $2-CClF_2$ | |
| 36 | 2 | 0 | H | 5- | $2-CBrF_2$ | |
| 37 | 2 | 0 | H | 5- | 2-SCN | |

Beispiel 3

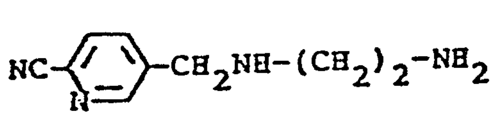

(Verbindung Nr. II-19)

Eine Lösung von 2-Cyano-5-pyridylmethylchlorid (4,6 g) in Acetonitril (20 ml) wurde tropfenweise bei 5 °C bis 10 °C zu einer Lösung von Ethylendiamin (9 g) in Acetonitril (50 ml) hinzugefügt. Nach der Zugabe wurde die Mischung 3 h bei Raumtemperatur gerührt. Acetonitril und der Überschuß des Ethylendiamins wurden unter vermindertem Druck aus der Mischung abdestilliert. Der Rückstand wurde mit Dichloromethan versetzt, und der in Dichloromethan lösliche Anteil wurde isoliert. Das Dichloromethan wurde unter vermindertem Druck abdestilliert, und flüchtige Stoffe wurden bei 50 °C und 1,33 mbar (1 mmHg) entfernt, wonach N-(2-Cyano-5-pyridylmethyl)ethylendiamin (4,5 g) als farbloses Öl erhalten wurde; $n_D^{20}$ : 1,5718.

Beispiel 4

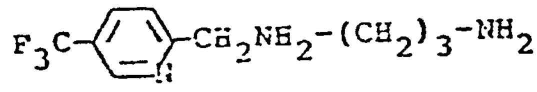

(Verbindung Nr. II-34)

5-Trifluoromethylpicolinaldehyd (3,5 g) wurde tropfenweise bei Raumtemperatur zu einer Lösung von Trimethylendiamin (7,4 g) in Benzol (70 ml) hinzugefügt. Nach der Zugabe wurde die Mischung allmählich unter Rühren erhitzt und dann 2 h unter Rückfluß gehalten, wobei Wasser azeotrop entfernt wurde. Das Benzol wurde unter vermindertem Druck abdestilliert. Der Rückstand wurde in Ethanol (100 ml) gelöst, und Natriumborhydrid (0,9 g) wurde nach und nach hinzugefügt, während die Lösung bei 10 °C bis 15 °C gerührt wurde. Die Mischung wurde dann 2 h bei Raumtemperatur gerührt, und Ethanol wurde bei weniger als 30 °C abdestilliert. Dem Rückstand wurde Dichloromethan zugegeben, und der in Dichloromethan lösliche Anteil wurde isoliert. Das Dichloromethan wurde unter vermindertem Druck abdestilliert, und flüchtige Stoffe wurden bei 1,33 mbar (1 mmHg) und weniger als 60 °C entfernt, wonach N-(5-Trifluoromethyl-2-pyridylmethyl)trimethylendiamin (3,5 g) als farbloses Öl erhalten wurde; $n_D^{20}$ : 1,4651.

Tabelle 2 zeigt speziell Verbindungen der Formel (II), die nach den gleichen Methoden wie in Beispiel 3 oder 4 hergestellt wurden.

Tabelle 2

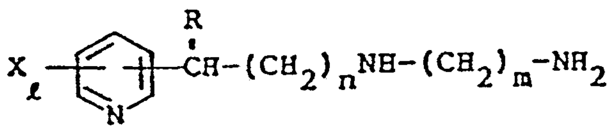

| Verbindung Nr. | m | n | R | Bindungsposition am Pyridin-Ring | $X_\ell$ mit Bindungsposition am Pyridinring | |
|---|---|---|---|---|---|---|
| II-1 | 3 | 0 | H | 5- | 2-CN | |
| II-2 | 2 | 0 | H | 5- | $2\text{-}OCH_2CF_3$ | |
| II-3 | 2 | 0 | H | 5- | $2\text{-}OCH_2F$ | |
| II-4 | 2 | 0 | H | 5- | $2\text{-}OCHF_2$ | |
| II-5 | 2 | 0 | H | 5- | $2\text{-}OCF_3$ | |
| II-6 | 3 | 0 | H | 5- | $2\text{-}OCF_3$ | |
| II-7. | 2 | 0 | $-CH_3$ | 5- | $2\text{-}OCF_3$ | |
| II-8 | 3 | 0 | H | 5- | $2\text{-}OCH_2CH_2F$ | |
| II-9 | 2 | 0 | H | 5- | $2\text{-}OCH_2CH_2Cl$ | |
| II-10 | 2 | 0 | H | 5- | $2\text{-}OCH_2CH_2Br$ | |
| II-11 | 3 | 0 | H | 5- | $2\text{-}OCH_2CF_3$ | |
| II-12 | 2 | 0 | H | 5- | $2\text{-}OCF_2CHF_2$ | |
| II-13 | 2 | 0 | H | 5- | $2\text{-}OCH(CF_3)_2$ | |
| II-14 | 3 | 0 | H | 5- | $2\text{-}SCF_3$ | |
| II-15 | 2 | 0 | H | 5- | $2\text{-}SCF_3$ | |
| II-16 | 2 | 1 | H | 5- | $2\text{-}SCH_2CF_3$ | |
| II-17 | 2 | 0 | H | 2- | $5\text{-}SCF_3$ | |
| II-18 | 2 | 0 | H | 5- | $2\text{-}SCH_2CH_2F$ | |
| II-20 | 2 | 0 | $-CH_3$ | 5- | 2-CN | |

Tabelle 2 - Fortsetzung

| Verbindung Nr. | m | n | R | Bindungsposition am Pyridin-Ring | $X_\ell$ mit Bindungsposition am Pyridin-Ring | |
|---|---|---|---|---|---|---|
| II-21 | 2 | 0 | H | 5- | $2-CH_2F$ | |
| II-22 | 2 | 0 | H | 5- | $2-CHF_2$ | |
| II-23 | 3 | 0 | H | 5- | $2-CHF_2$ | |
| II-24 | 2 | 0 | H | 5- | $2-CF_3$ | $n_D^{20}$ 1.4811 |
| II-25 | 3 | 0 | H | 5- | $2-CF_3$ | |
| II-26 | 2 | 0 | $-CH_3$ | 5- | $2-CF_3$ | |
| II-27 | 2 | 0 | H | 2- | $5-CF_3$ | |
| II-28 | 2 | 0 | H | 5- | $2-CH_2CH_2F$ | |
| II-29 | 2 | 0 | H | 5- | $2-CH_2CH_2Cl$ | |
| II-30 | 2 | 0 | H | 5- | $2-CH_2CF_3$ | |
| II-31 | 2 | 0 | H | 5- | $2-CF_2CHF_2$ | |
| II-32 | 2 | 0 | H | 5- | $2-CF_2CF_3$ | |
| II-33 | 2 | 1 | H | 5- | $2-CF_3$ | |
| II-35 | 2 | 0 | H | 5- | $2-CClF_2$ | |
| II-36 | 2 | 0 | H | 5- | $2-CBrF_2$ | |
| II-37 | 2 | 0 | H | 5- | 2-SCN | |

Beispiel 5

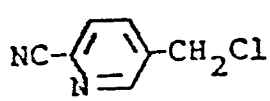

2-Cyano-5-hydroxymethylpyridin (13,4 g) und Pyridin (8,7 g) wurden in Toluol (150 ml) gelöst, und eine Lösung von p-Toluolsulfonylchlorid (19,1 g) in Toluol (50 ml) wurde tropfenweise bei Raumtemperatur zu der Lösung hinzugefügt. Nach der Zugabe wurde die Mischung allmählich erhitzt und 4 h bei 70° C bis 80° C gerührt. Die Reaktionsmischung wurde auf Raumtemperatur abgekühlt und mit Wasser gewaschen. Die organische Schicht wurde getrocknet. Das Toluol wurde unter vermindertem Druck abdestilliert. Der Rückstand wurde durch Silicagel-Säulenchromatographie gereinigt, wonach 2-Cyano-5-pyridylmethylchlorid (7,7 g) in Form farbloser Kristalle erhalten wurde; Schmp. 45-47° C.

Beispiel 6

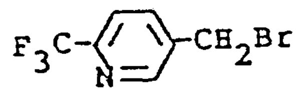

5-Methyl-2-trifluoromethylpyridin (siehe J. Org. Chem. 29, Seiten 569-571) (8,1 g) wurde in 50 ml Kohlenstofftetrachlorid gelöst, und N-Bromosuccinimid (8,9 g) sowie eine katalytische Menge Benzoylperoxid wurden zugegeben. Die Mischung wurde 7 h unter Rühren zum Rückfluß erhitzt. Die Reaktionsmischung wurde abgekühlt, und unlösliche Stoffe wurden durch Filtration gesammelt. Das Filtrat wurde unter vermindertem Druck konzentriert, und der Rückstand wurde durch Silicagel-Säulenchromatographie gereinigt, wonach 2-Trifluoromethyl-5-pyridylmethylbromid (auch als 5-Bromomethyl-2-trifluoromethylpyridin bekannt) (7,3 g) erhalten wurde. Schmp. 36-37° C.

Biologische Tests:

Vergleichs-Verbindung A-1:

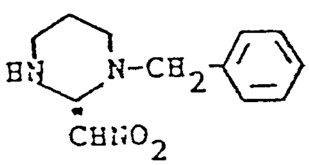

die in der DE-OS 25 14 402 beschriebene Verbindung. Diese Verbindung diente als Vergleichs-Verbindung in den folgenden Beispielen 7, 8 und 9.

Beispiel 7 (Biologischer Test)

Test mit gegen Organophosphor-Mittel resistenten Nephotettix cincticeps:

Herstellung einer Test-Chemikalie:

| | | |
|---|---|---|
| Lösungsmittel: | 3 Gew.-Teile | Xylol |
| Emulgator: | 1 Gew.-Teil | Polyoxyethylen-alkylphenylether |

Zur Herstellung eines geeigneten Test-Präparats wurde 1 Gew.-Teil der aktiven Verbindung mit der oben bezeichneten Menge Lösungsmittel, das die oben angegebene Menge Emulgator enthielt, vermischt, und die Mischung wurde mit Wasser auf eine vorher festgesetzte Konzentration verdünnt.

Test-Verfahren:

Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Konzentration jeder der aktiven Verbindungen aufweisenden Lösungen, die wie oben angegeben hergestellt wurden, gesprüht. Die aufgesprühte Chemikalie wurde trocknen gelassen, und über die Reispflanzen wurde ein Drahtkorb von 7 cm Durchmesser und 14 cm Höhe gestülpt, unter dem 30 ausgewachsene weibliche Exemplare von Nephotettix cincticeps, die gegen Organophosphor-Insektizide resistent waren, ausgesetzt wurden. Die Töpfe wurden jeweils in einem Raum mit konstanter Temperatur aufbewahrt, und 2 Tage später wurde die Zahl der toten Insekten bestimmt und das Vernichtungsverhältnis berechnet.

In diesem Test zeigten beispielsweise die folgenden Verbindungen aus den Herstellungsbeispielen eine überlegene Wirkung im Vergleich zum Stand der Technik: Verbindungen Nr. 1 und Nr. 2.

Beispiel 8 (Biologischer Test)

Test an Laternenträgern

Test-Verfahren:

Eine mit Wasser verdünnte, eine vorbestimmte Wirkstoff-Konzentration aufweisende Lösung, die wie in Beispiel 9 hergestellt worden war, wurde in einer Menge von 10 ml pro Topf auf Reispflanzen von etwa 10 cm Höhe gesprüht, die in Töpfen von 12 cm Durchmesser gezogen worden waren. Die aufgesprühte Chemikalie wurde trocknen gelassen, und über jeden Topf wurde ein Drahtkorb von 7 cm Durchmesser und 14 cm Höhe gestülpt. 30 ausgewachsene weibliche Exemplare von Nilaparvata lugens Stael eines Stammes, der Resistenz gegen Organophosphor-Chemikalien zeigte, wurden unter jedem Drahtkorb ausgesetzt. Die Töpfe wurden jeweils in einem Raum mit konstanter Temperatur aufbewahrt, und 2 Tage später wurde die Zahl der toten Insekten bestimmt. Das Vernichtungsverhältnis wurde berechnet.

In der gleichen Weise wurde das Vernichtungsverhältnis berechnet für Sogatella furcifera Horvath und organophosphorresistente Laodelphax striatellus Fallen.

In diesem Test zeigten beispielsweise die folgenden Verbindungen aus den Herstellungsbeispielen eine überlegene Wirkung im Vergleich zum Stand der Technik: Verbindungen Nr. 1, 2, 11, 19 und 24.

Beispiel 9 (Biologischer Test)

Test mit gegen Organophosphor- und Carbamat-Chemikalien resistentem Myzus persicae (grüne Pfirsichblattlaus):

Test-Verfahren:

Gezüchtete grüne Pfirsichblattläuse, die gegen Organophosphor-Chemikalien und Carbamat-Chemikalien resistent waren, wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 9 hergestellte wäßrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28° C stehen gelassen. 24 Stunden nach dem Sprühen wurde das Vernichtungsverhältnis berechnet. Für jede Verbindung wurde der Test als Doppelbestimmung durchgeführt.

In diesem Test zeigten beispielsweise die nachstehend bezeichneten Verbindungen aus den Herstellungsbeispielen eine überlegene Wirkung im Vergleich zum Stand der Technik: Verbindungen Nr. 2, Nr. 11 und Nr.12.

Die Beispiele 7, 8 und 9 stellen typische Beispiele für insektizide Anwendungen dar, und die in ihnen gezeigten Verbindungen sind typische Beispiele. Es ist jedoch ausdrücklich festzustellen, daß die Erfindung nicht allein auf diese Beispiele beschränkt ist.

## Patentansprüche

**1.** Nitromethylen-Derivate der Formel (I)

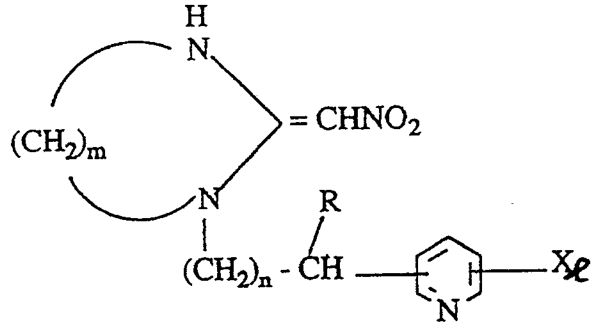

(I)

in der

R ein Wasserstoff-Atom oder eine Alkyl-Gruppe mit 1 bis 4 Kohlenstoff-Atomen ist,

X eine durch Fluor, Chlor und/oder Brom substituierte $C_1$-$C_4$-Alkyl-Gruppe, eine durch Fluor und/oder Chlor substituierte $C_1$-$C_4$-Alkoxy-Gruppe, eine durch Fluor und/oder Chlor substituierte $C_1$-$C_4$-Alkylthio-Gruppe, eine Cyano-Gruppe oder eine Thiocyanato-Gruppe ist,
ℓ 1 oder 2 ist,
m 2 oder 3 ist und
n 0 ist.

**2.** Verbindungen nach Anspruch 1, dadurch gekennzeichnet, daß
R ein Wasserstoff-Atom oder eine Methyl-Gruppe ist,
X eine durch Fluor substituierte $C_1$-$C_2$-Alkyl-Gruppe, eine durch Fluor substituierte $C_1$-$C_2$-Alkoxy-Gruppe, eine durch Fluor substituierte $C_1$-$C_2$-Alkylthio-Gruppe, eine Cyano-Gruppe oder eine Thiocyanato-Gruppe ist,
ℓ 1 oder 2 ist,
m 2 oder 3 ist und
n 0 ist.

**3.** Verbindungen nach Ansprüchen 1 bis 3, ausgewählt aus 1-(2-Trifluoromethyl-5-pyridylmethyl)-2-(nitromethylen)imidazolidin der Formel

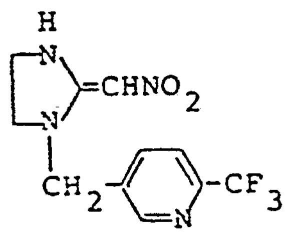

und
1-[2-(2,2,2-Trifluoroethoxy)-5-pyridylmethyl]-2-(nitromethylen)imidazolidin der Formel

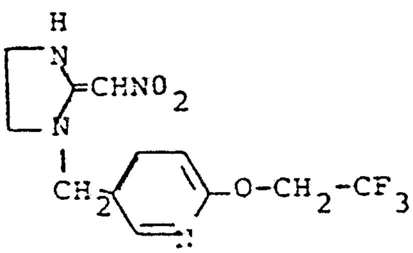

**4.** Verfahren zur Herstellung von Nitromethylen-Derivaten der Formel (I)

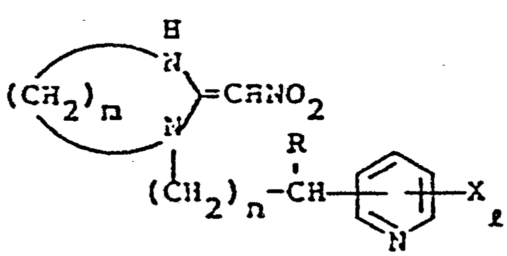

(I)

in der
R ein Wasserstoff-Atom oder eine Alkyl-Gruppe mit 1 bis 4 Kohlenstoff-Atomen ist,
X eine durch Fluor, Chlor und/oder Brom substituierte $C_1$-$C_4$-Alkyl-Gruppe, eine durch Fluor und/oder Chlor substituierte $C_1$-$C_4$-Alkoxy-Gruppe, eine durch Fluor und/oder Chlor substitu-

ierte $C_1$-$C_4$-Alkylthio-Gruppe, eine Cyano-Gruppe oder eine Thiocyanato-Gruppe ist,

$\ell$ 1 oder 2 ist,

m 2 oder 3 ist und

n 0 ist,

dadurch gekennzeichnet, daß

die Verbindungen der Formel (II)

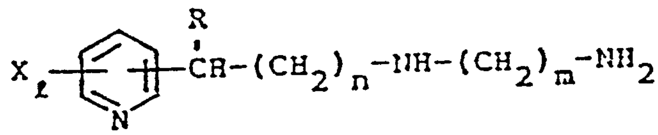 (II) ,

in der R, X, $\ell$, m und n die im Vorstehenden angegebenen Bedeutungen haben, mit den Verbindungen der Formel (III)

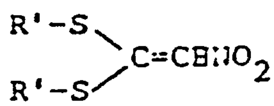 (III) ,

in der

R' eine niedere Alkyl-Gruppe oder eine Benzyl-Gruppe bezeichnet oder die beiden Gruppe R' zusammengenommen eine niedere Alkylen-Gruppe mit wenigstens zwei Kohlenstoff-Atomen bezeichnen und zusammen mit den ihnen benachbarten Schwefel-Atomen einen Ring bilden können,

in Gegenwart inerter Lösungsmittel umgesetzt werden,

oder

die Verbindungen der Formel (IV)

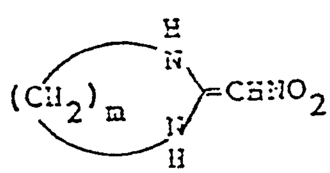 (IV) ,

in der m die im Vorstehenden angegebenen Bedeutungen hat, mit den Verbindungen der Formel (V)

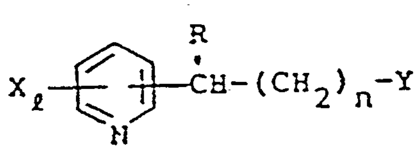 (V) ,

in der

R, X, $\ell$ und n die im Vorstehenden angegebenen Bedeutungen haben und

Y ein Halogen-Atom oder eine Gruppe der Formel $-OSO_2R''$ bezeichnet, worin R'' für eine niedere Alkyl-Gruppe oder eine Aryl-Gruppe steht,

in Gegenwart inerter Lösungsmittel, gegebenenfalls in Anwesenheit von Basen, umgesetzt werden.

**5.** Insektizide Mittel, dadurch gekennzeichnet, daß sie wenigstens eines der Nitromethylen-Derivate der Formel (I) enthalten.

**6.** Verfahren zur Bekämpfung von Schadinsekten, dadurch gekennzeichnet, daß man Nitromethylen-Derivate der Formel (I) auf die schädlichen Insekten und/oder deren Lebensraum einwirken läßt.

7. Verwendung von Nitromethylen-Derivaten der Formel (I) zur Bekämpfung von Schadinsekten.

8. Verfahren zur Herstellung von insektiziden Mitteln, dadurch gekennzeichnet, daß Nitromethylen-Derivate der Formel (I) mit Streckmitteln und/oder grenzflächenaktiven Mitteln vermischt werden.

## Claims

1. Nitromethylene derivatives of the formula (I)

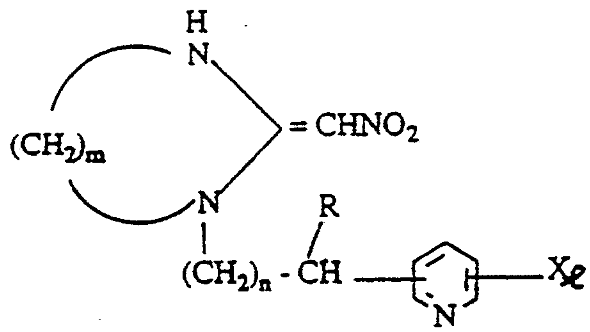

(I)

in which

- R is a hydrogen atom or an alkyl group having 1 to 4 carbon atoms,
- X is a $C_1$-$C_4$-alkyl group which is substituted by fluorine, chlorine and/or bromine, a $C_1$-$C_4$-alkoxy group which is substituted by fluorine and/or chlorine, a $C_1$-$C_4$-alkylthio group which is substituted by fluorine and/or chlorine, a cyano group or a thiocyanato group,
- l is 1 or 2,
- m is 2 or 3 and
- n is 0.

2. Compounds according to Claim 1, characterised in that

- R is a hydrogen atom or a methyl group,
- X is a $C_1$-$C_2$-alkyl group which is substituted by fluorine, a $C_1$-$C_2$-alkoxy group which is substituted by fluorine, a $C_1$-$C_2$-alkylthio group which is substituted by fluorine, a cyano group or a thiocyanato group,
- l is 1 or 2,
- m is 2 or 3 and
- n is 0.

3. Compounds according to Claims 1 to 3, selected from amongst 1-(2-trifluoromethyl-5-pyridylmethyl)-2-(nitromethylene)imidazolidine of the formula

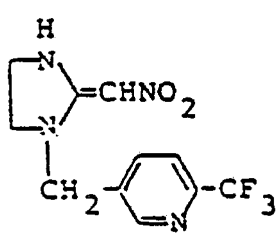

and

1-[2-(2,2,2-trifluoroethoxy)-5-pyridylmethyl]-2-(nitromethylene)imidazolidine of the formula

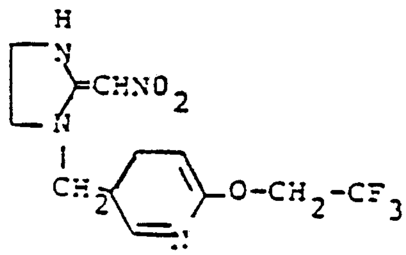

4. Process for the preparation of nitromethylene derivatives of the formula (I)

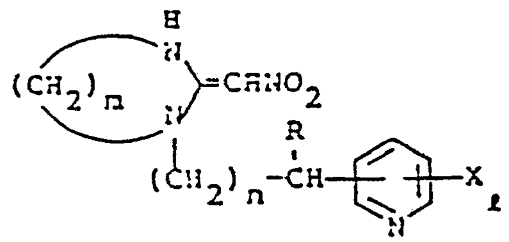

(I)

in which

- R is a hydrogen atom or an alkyl group having 1 to 4 carbon atoms,
- X is a $C_1$-$C_4$-alkyl group which is substituted by fluorine, chlorine and/or bromine, a $C_1$-$C_4$-alkoxy group which is substituted by fluorine and/or chlorine, a $C_1$-$C_4$-alkylthio group which is substituted by fluorine and/or chlorine, a cyano group or a thiocyanato group,
- l is 1 or 2,
- m is 2 or 3 and
- n is 0,

characterised in that
the compounds of the formula (II)

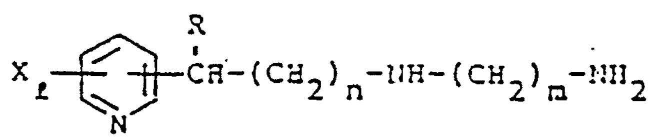

(II)

in which R, X, l, m and n have the meanings mentioned above, are reacted with the compounds of the formula (III)

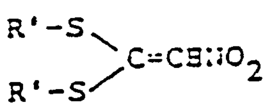

(III)

in which

- R' denotes a lower alkyl group or a benzyl group, or the two R' groups together denote a lower alkylene group having at least two carbon atoms and can, together with the adjacent sulphur atoms, form a ring,

in the presence of inert solvents,
or
the compounds of the formula (IV)

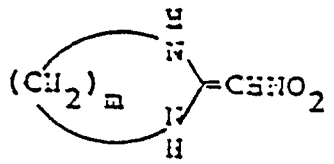

(IV)

in which m has the meanings indicated above, are reacted with the compounds of the formula (V)

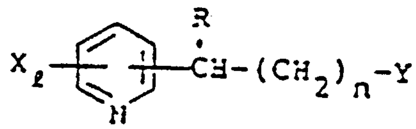

(V)

in which

- R, X, l and n — have the meanings indicated above and
- Y — denotes a halogen atom or a group of the formula $-OSO_2R''$, in which R'' represents a lower alkyl group or an aryl group,

in the presence of inert solvents, if appropriate in the presence of bases.

**5.** Insecticidal agents, characterised in that they contain at least one of the nitromethylene derivatives of the formula (I).

**6.** Method of controlling insect pests, characterised in that nitromethylene derivatives of the formula (I) are allowed to act on the harmful insects and/or their environment.

**7.** Use of nitromethylene derivatives of the formula (I) for controlling insect pests.

**8.** Process for the preparation of insecticidal agents, characterised in that nitromethylene derivatives of the formula (I) are mixed with extenders and/or surface-active agents.

**Revendications**

**1.** Dérivés nitrométhyléniques de formule (I)

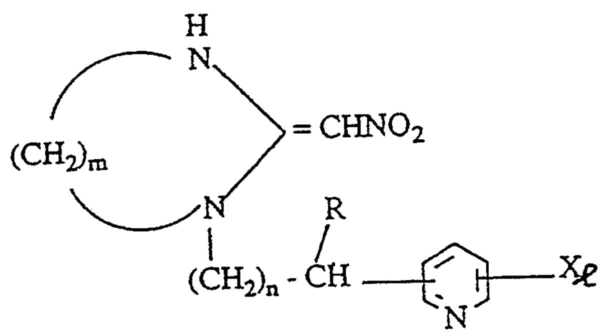

(I)

dans laquelle

- R — est un atome d'hydrogène ou un groupe alkyle ayant 1 à 4 atomes de carbone,
- X — est un groupe alkyle en $C_1$ à $C_4$ substitué par du fluor, du chlore et/ou du brome, un groupe alkoxy en $C_1$ à $C_4$ substitué par du fluor et/ou du chlore, un groupe alkylthio en $C_1$ à $C_4$ substitué par du fluor et/ou du chlore, un groupe cyano ou un groupe thiocyanato,
- $\underline{\ell}$ — a la valeur 1 ou 2,
- $\underline{m}$ — a la valeur 2 ou 3, et
- $\underline{n}$ — est égal à 0.

**2.** Composés suivant la revendication 1, caractérisés en ce que

- R est un atome d'hydrogène ou un groupe méthyle,
- X est un groupe alkyle en $C_1$ ou $C_2$ substitué par du fluor, un groupe alkoxy en $C_1$ ou $C_2$ substitué par du fluor, un groupe alkylthio en $C_1$ ou $C_2$ substitué par du fluor, un groupe cyano ou un groupe thiocyanato,
- $\ell$ a la valeur 1 ou 2,
- $m$ a la valeur 2 ou 3, et
- $n$ est égal à 0.

**3.** Composés suivant les revendications 1 à 3, choisis entre la 1-(2-trifluorométhyl-5-pyridylméthyl)-2-(nitrométhylène)imidazolidine de formule

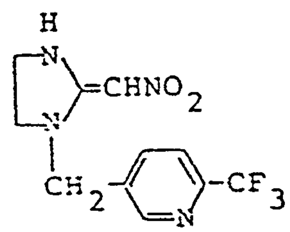

et
la 1-[2-(2,2,2-trifluoréthoxy)-5-pyridylméthyl]-2-(nitrométhylène)imidazolidine de formule

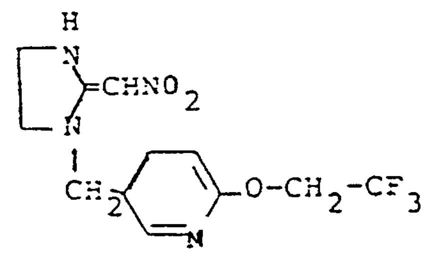

**4.** Procédé de production de dérivés nitrométhyléniques de formule (I)

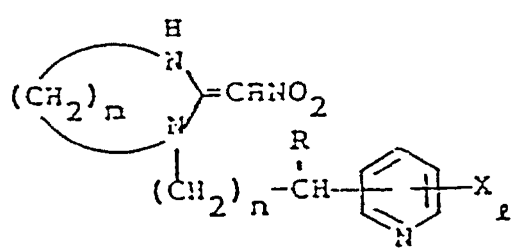
(I)

dans laquelle
- R est un atome d'hydrogène ou un groupe alkyle ayant 1 à 4 atomes de carbone,
- X est un groupe alkyle en $C_1$ à $C_4$ substitué par du fluor, du chlore et/ou du brome, un groupe alkoxy en $C_1$ à $C_4$ substitué par du fluor et/ou du chlore, un groupe alkylthio en $C_1$ à $C_4$ substitué par du fluor et/ou du chlore, un groupe cyano ou un groupe thiocyanato,
- $\ell$ a la valeur 1 ou 2,
- $m$ a la valeur 2 ou 3, et
- $n$ est égal à 0,

caractérisé en ce qu'on fait réagir en présence de solvants inertes les composés de formule (II)

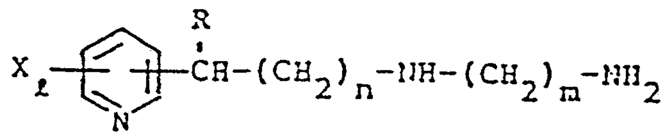
(II)

dans laquelle R, X, $\ell$, m et n ont les définitions indiquées précédemment, avec les composés de formule (III)

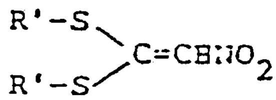
(III)

dans laquelle

R' est un groupe alkyle inférieur ou un groupe benzyle ou les deux groupes R' pris conjointement représentent un groupe alkylène inférieur ayant au moins 2 atomes de carbone et peuvent former un noyau conjointement avec les atomes de soufre qui leur sont contigus,

ou bien

on fait réagir en présence de solvants inertes, éventuellement en présence de bases, les composés de formule (IV)

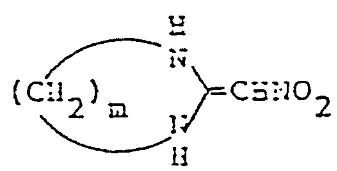
(IV)

dans laquelle m a les définitions indiquées précédemment, avec les composés de formule (V)

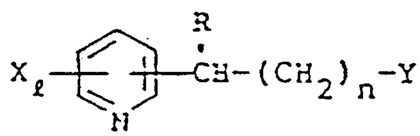
(V)

dans laquelle

R, X, $\ell$ et n ont les définitions indiquées précédemment et

Y est un atome d'halogène ou un groupe de formule $-OSO_2R''$ dans laquelle R'' représente un groupe alkyle inférieur ou un groupe aryle.

**5.** Compositions insecticides, caractérisées en ce qu'elles contiennent au moins l'un des dérivés nitrométhyléniques de formule (I).

**6.** Procédé pour combattre des insectes parasites, caractérisé en ce qu'on fait agir des dérivés nitrométhyléniques de formule (I) sur les insectes parasites et/ou sur leur habitat.

**7.** Utilisation de dérivés nitrométhyléniques de formule (I) pour combattre des insectes parasites.

**8.** Procédé de préparation de compositions insecticides, caractérisé en ce qu'on mélange des dérivés nitrométhyléniques de formule (I) avec des diluants et/ou des agents tensio-actifs.